# EUROPEAN PATENT APPLICATION

(11) **EP 4 349 866 A1**
(43) Date of publication of application: **10.04.2024**
(21) Application number: 22811711.5
(22) Date of filing: 26.05.2022
(51) Int. Cl.: C07K 16/40, G01N 33/68, C12N 15/85

(54) **ANTIBODY SPECIFICALLY BINDING TO ASM PROTEIN**

(30) Priority: 27.05.2021 KR 20210068075; 18.04.2022 KR 20220047295
(71) Applicant: Isu Abxis Co., Ltd., Seongnam-si, Gyeonggi-do 13488 (KR)
(72) Inventor: HONG, Seung Beom, Gwangju-si Gyeonggi-do 12784 (KR); HONG, Mi Rim, Seoul 04358 (KR)
(74) Representative: Maiwald GmbH
(86) International application number: PCT/KR2022/095105
(87) International publication number: WO 2022/250520

(57) **Abstract**

The present disclosure relates to an antibody specifically binding to an acid sphingomyelinase (ASM) protein and, specifically, the antibody or antigen-binding fragment according to the present disclosure specifically binds to an ASM protein with high affinity and thus can be advantageously used in detecting the ASM protein or diagnosing a disease occurring due to the over-expression of the ASM protein.

## Description

### 1. Field of the invention

The present disclosure relates to antibodies specifically binding to acid sphingomyelinase (ASM).

### 2. Description of the Prior Art

Sphingolipid metabolism regulates normal cellular signaling, and abnormal changes in sphingolipid metabolism cause various neurodegenerative diseases including Alzheimer's disease. Acid sphingomyelinase (ASM) protein, an enzyme that regulates sphingolipid metabolism, is a protein expressed in almost all types of cells and plays an important role in sphingolipid metabolism and cell membrane turnover.

In the brain of patients with neurodegenerative diseases such as Alzheimer's disease, the activity of ASM protein is significantly increased compared with that of normal people. In this connection, Korean Patent No. 10-1521117 discloses that the inhibition of the activity of over-expressed ASM protein or the inhibition of the expression into ASM protein suppresses the accumulation of amyloid-β and improves learning ability and memory and thus can treat neurodegenerative diseases. It has been recently known that the activity of ASM protein is also increased in neurological diseases, such as depression, and thus the inhibition of the expression or activity of ASM protein is effective in the amelioration of depression.

However, substances that directly inhibit the expression or activity of ASM protein have not been developed, and several types of inhibitors that indirectly inhibit the expression of ASM proteins have been identified. For example, there are tricyclic antidepressants used in the treatment of depression, and examples thereof include amitriptyline, desipramine, mipramine and the like. Although these tricyclic antidepressants were not developed as ASM protein inhibitors, various studies have demonstrated that such antidepressants exhibit ASM protein inhibitory effects. A major pharmacological mechanism of tricyclic antidepressants is that the activity thereof is increased by inhibiting the reuptake of neurotransmitters in nerve cells, and their action as an ASM inhibitor was confirmed to be a subordinate action. However, tricyclic antidepressants may act on the nervous system and nerve cells, causing side effects, such as blurred vision, increased light sensitivity, and vomiting.

### SUMMARY OF THE INVENTION

Therefore, an aspect of the present disclosure is to provide an antibody or antigen-binding fragment thereof that specifically binds to ASM protein.

Another aspect of the present disclosure is to provide a method for producing the antibody or antigen-binding fragment thereof.

Still another aspect of the present disclosure is to provide use of the antibody or antigen-binding fragment thereof for detecting ASM protein.

In accordance with aspects, the present disclosure provides an antibody or antigen-binding fragment thereof that specifically binds to an acid sphingomyelinase (ASM) protein.

Furthermore, the present disclosure provides a nucleic acid encoding the antibody or antigen-binding fragment thereof.

Furthermore, the present disclosure provides an expression vector including the nucleic acid.

Furthermore, the present disclosure provides a host cell including the nucleic acid or the expression vector.

Furthermore, the present disclosure provides a method for producing an antibody or antigen-binding fragment thereof that specifically binds to an ASM protein, the method including culturing the host cell to produce the antibody or antigen-binding fragment thereof.

Furthermore, the present disclosure provides a composition and kit for detecting an ASM protein, each including the antibody or antigen-binding fragment thereof.

Furthermore, the present disclosure provides a method for detecting an ASM protein, the method including reacting a sample with the antibody or antigen-binding fragment thereof.

The antibody or antigen-binding fragment according to the present disclosure specifically binds to an ASM protein with high affinity and thus can be advantageously used in detecting the ASM protein or diagnosing a disease occurring due to the over-expression of the ASM protein.

### BRIEF DESCRIPTION OF THE DRAWINGS

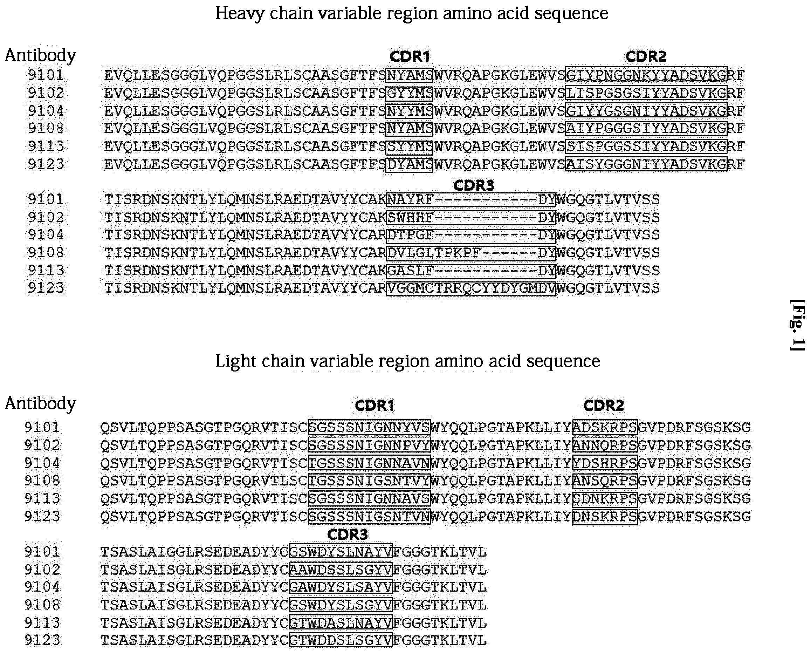

FIG. 1 illustrates a schematic diagram showing amino acid sequences of heavy and light chain variable regions of antibodies produced according to an example of the present disclosure.
FIG. 2 illustrates a graph showing the binding of the antibodies produced according to an example of the present disclosure to ASM protein, as confirmed by ELISA analysis.
FIG. 3 illustrates a schematic diagram showing the antigen binding sites of the antibodies produced according to an example of the present disclosure on ASM protein, in an ASM protein structure model.
FIG. 4 illustrates a deuterium exchange heat map, in the ASM protein saposin domain, of each of the antibodies produced according to an example of the present disclosure.

### DETAILED DESCRIPTION OF THE EXEMPLARY EMBODIMENTS

Hereinafter, the present disclosure will be described in detail.

The present disclosure provides an antibody or antigen-binding fragment thereof that specifically binds to an acid sphingomyelinase (ASM) protein.

As used herein, the term "acid sphingomyelinase (ASM) protein" refers to an enzyme, which is a member of the sphingomyelinase (SMase) family that regulates sphingolipid metabolism. The ASM protein catalyzes the degradation of sphingomyelin into ceramide and phosphorylcholine, and may be classified as an alkaline, neutral, or acidic ASM protein depending to pH at which optimal enzymatic activity thereof is exhibited.

The ASM protein may encompass any type of ASM protein that is known in the art. Specifically, the ASM protein may be derived from a mammal, more specifically, a human, a monkey, a rat, or a mouse. In addition, the ASM protein may contain all the amino acid sequences known as the ASM protein in the art. For example, the ASM protein may be a polypeptide consisting of the amino acid sequences as set forth in SEQ ID NO: 66 and 67, respectively, or a nucleic acid encoding the same.

The ASM protein may be one that has addition, deletion, or substitution of at least one amino acid in the amino acid sequence as set forth in SEQ ID NO: 66 or 67 as long as one retains biological activity equivalent or corresponding to that of the ASM protein. The substitution of the amino acid may be conservative substitution that is performed within a range in which the substitution does not affect or gives a weak effect on the entire protein charge, that is, polarity or hydrophobicity. The ASM protein may also have at least 80%, at least 90%, at least 95%, at least 97%, or at least 99% homology with the amino acid sequences as set forth in SEQ ID NO: 66 and 67, respectively. The ASM protein may contain only a portion thereof as long as the portion retains biological activity equivalent or corresponding to that of the ASM protein.

As used herein, the term "antibody" refers to an immune protein that binds to an antigen to interfere with an action of the antigen or eliminate the antigen. The antibody may include all types of antibodies known in the art. Specifically, the antibody may include IgM, IgD, IgG, IgA, and IgE, which may contain heavy chains formed from µ, δ, γ, α, and ε genes encoding heavy chain constant regions, respectively. In antibody technology, IgG is typically used. IgG may include an isotype, IgG1, IgG2, IgG3, or IgG4, which may be different in terms of structural and functional characteristics. The antibody may include all of a humanized antibody containing a minimal sequence derived from a non-human antibody, a human antibody containing a sequence derived from a human, or a chimeric antibody containing mixed sequences derived from different species.

The IgG may have a very stable Y-shaped structure (about 150 kDa) made of two heavy chain proteins of about 50 kDa and two light chain proteins of about 25 kDa. Heavy and light chains constituting antibodies may be divided into variable regions having amino acid sequences, which are different among antibodies, and constant regions having amino acid sequences, which are same among antibodies. The heavy chain constant regions include CH1, hinge (H), CH2, and CH3 domains, each of which is composed of two β-sheets and which may be linked via an intermolecular disulfide bond. In addition, the light chain constant regions may include a CL domain. Two heavy chain and light chain variable regions are combined to form an antigen-binding site, and one antigen binding site may be present in each of the two Y-shaped arms. In the full-length antibody, a region that can bind to an antigen is called an antibody binding fragment (Fab), and a region that cannot bind to an antigen is called a crystallizable fragment (Fc), and the Fab and Fc may be connected by a hinge. In an embodiment of the present disclosure, the IgG may be a mouse-derived IgG and, specifically, may include a mouse IgG heavy chain constant region consisting of the amino acid sequence as set forth in SEQ ID NO: 74 and a mouse light chain λ constant region consisting of the amino acid sequence as set forth in SEQ ID NO: 76. Alternatively, the mouse-derived IgG may include a mouse IgG heavy chain constant region consisting of the nucleotide sequence as set forth in SEQ ID NO: 75 and a mouse light chain λ constant region containing the nucleotide sequence as set forth in SEQ ID NO: 77.

The antibody according to the present disclosure may include not only the full-length antibody but also an antigen-binding fragment thereof. Specifically, the antigen-binding fragment may refer to a region except for Fc that functions to transmit the stimulus with an antigen to a cell, a complement, or the like. For example, the antigen-binding fragment of the antibody may include all of Fab, scFv, F(ab)2, and Fv, and may also include a 3rd generation antibody fragment, such as a single domain antibody and a minibody.

In an aspect of the present disclosure, the antibody or antigen-binding fragment may bind to at least one epitope that is selected from the group consisting of a fragment of amino acids at positions 53 to 72, a fragment of amino acids at positions 101 to 123, a fragment of amino acids at positions 135 to 159, a fragment of amino acids at positions 135 to 155, a fragment of amino acid at positions 218 to 228, and a fragment of amino acids at positions 259 to 269 from the N-terminus of the ASM protein. The ASM protein may have the features as described above. In an embodiment of the present disclosure, the fragment of amino acids at positions 53 to 72 from the N-terminus of the ASM protein may be a polypeptide consisting of the amino acid sequence as set forth in SEQ ID NO: 68 (TAINLGLKKEPNVARVGSVA); the fragment of amino acids at positions 101 to 123 from the N-terminus of the ASM protein may be a polypeptide consisting of the amino acid sequence as set forth in SEQ ID NO: 69 (VWRRSVLSPSEACGLLLGSTCGH); the fragment of amino acids at positions 135 to 159 from the N-terminus of the ASM protein may be a polypeptide consisting of the amino acid sequence as set forth in SEQ ID NO: 70 (PTVPKPPPKPPSPPAPGAPVSRILF); the fragment of amino acids at positions 135 to 155 from the N-terminus of the ASM protein may be a polypeptide consisting of the amino acid sequence as set forth in SEQ ID NO: 71 (PTVPKPPPKPPSPPAPGAPVS); the fragment of amino acids at positions 218 to 228 from the N-terminus of the ASM protein may be a polypeptide consisting of the amino acid sequence as set forth in SEQ ID NO: 72 (SGLGPAGPFDM); and the fragment of amino acids at positions 259 to 269 from the N-terminus of the ASM protein may be a polypeptide consisting of the amino acid sequence as set forth in SEQ ID NO: 73 (VRKFLGPVPVY). That is, according to another aspect of the present disclosure, the antibody or antigen-binding fragment may bind to at least one epitope selected from the group consisting of the polypeptides sets forth in SEQ ID NOS: 68 to 73, respectively.

As used herein, the term "epitope" refers to a specific site of an antigen that can be recognized by an antibody, and means an antigenic determinant to which an antibody can specifically bind. The epitope is commonly used as the same meaning as a determinant or an antigenic determinant. The epitope consists of chemically active surface groupings of molecules, such as amino acids or sugar side chains, and has usually specific three-dimensional structural as well as specific charge characteristics.

In an aspect of the present disclosure, the antibody or antigen-binding fragment thereof may include: a heavy chain variable region comprising a heavy chain CDR1 (X₁YX₂MS) consisting of the amino acid sequence as set forth in SEQ ID NO: 61, a heavy chain CDR2 (X₃IX₄X₅X₆X₇X₈X₉X₁₀YYADSVKG) consisting of the amino acid sequence as set forth in SEQ ID NO: 62, and a heavy chain CDR3 selected from the group containing of heavy chain CDR2 consisting of amino acid sequences as set forth in SEQ ID NOS: 27, 30, 33, 36, 39, and 42; and a light chain variable region including a light chain CDR1 (X₁₁GSSSNIGX₁₂NX₁₃VX₁₄) consisting of the amino acid sequence as set forth in SEQ ID NO: 63, a light chain CDR2 (X₁₅X₁₆X₁₇X₁₈RPS) consisting of the amino acid sequence as set forth in SEQ ID NO: 64, and a light chain CDR3 (X₁₉X₂₀WDX₂₁SLX₂₂X₂₃YV) consisting of the amino acid sequence as set forth in SEQ ID NO 65.

The term "complementarity determining region (CDR)" refers to a hypervariable region that is in the heavy chain and light chain variable regions of an antibody and has a different amino acid sequence for each antibody, and means a region that actually binds to an antigen. In a three-dimensional conformation of an antibody, CDR has a loop shape on a surface of the antibody, wherein under the loop, a framework region (FR) may exist for structurally supporting CDR. There are three loop structures in each of the heavy chain and the light chain, and these six loop structures can be combined together to directly contact an antigen. The antigen-binding site having six loop structures, CDRs, may be, for convenience, heavy chain CDR1, heavy chain CDR2, heavy chain CDR3, light chain CDR1, light chain CDR2 or light chain CDR3.

For example, in the heavy chain CDR1 (X₁YX₂MS) consisting of the amino acid sequence as set forth in SEQ ID NO: 61, X₁ and X₂ each may be selected from acidic and neutral amino acids. Specifically, X₁ may be an acidic or neutral amino acid and X₂ may be a neutral amino acid. For example, X₁ and X₂ each may be selected from the group consisting of asparagine (Asn), glycine (Gly), serine (Ser), aspartic acid (Asp), alanine (Ala), and tyrosine (Tyr). Specifically, X₁ may be asparagine, glycine, serine, or aspartic acid, and X₂ may be alanine or tyrosine. In an embodiment of the present disclosure, the heavy chain CDR1 may consist of an amino acid sequence as set forth in SEQ ID NO: 25, 28, 31, 34, 37, or 40.

In the heavy chain CDR2 (X₃IX₄X₅X₆X₇X₈X₉X₁₀YYADSVKG) consisting of the amino acid sequence of SEQ ID NO: 62, X₃ to X₁₀ each may be selected from neutral and basic amino acids. Specifically, X₃ and X₄ to X₉ each may be a neutral amino acid, and X₁₀ may be a neutral or basic amino acid. For example, X₃ to X₁₀ each may be selected from the group consisting of glycine, leucine (Leu), alanine, serine, tyrosine, proline (Pro), asparagine, lysine (Lys), and isoleucine (Ile). Specifically, X₃ may be glycine, leucine, alanine, or serine; X₄ may be tyrosine or serine; X₅ may be proline or tyrosine; X₆ may be asparagine or glycine; X₇ and X₈ each may be glycine or serine; X₉ may be asparagine or serine; and X₁₀ may be lysine or isoleucine. In an embodiment of the present disclosure, the heavy chain CDR2 may consist of an amino acid sequence as set forth in SEQ ID NO: 26, 29, 32, 35, 38, or 41.

In the light chain CDR1 (X₁₁GSSSNIGX₁₂NX₁₃VX₁₄) consisting of the amino acid sequence as set forth in SEQ ID NO: 63, X₁₁ to X₁₄ each may be selected from neutral amino acids. For example, X₁₁ to X₁₄ each may be selected from the group consisting of threonine (Thr), serine, asparagine, alanine, proline, and tyrosine. Specifically, X₁₁ may be threonine or serine; X₁₂ may be asparagine or serine; X₁₃ may be alanine, proline, threonine, or tyrosine; and X₁₄ may be asparagine, tyrosine, or serine. In an embodiment of the present disclosure, the light chain CDR1 may consist of an amino acid sequence as set forth in SEQ ID NO: 43, 46, 49, 52, 55, or 58.

In the light chain CDR2 (X₁₅X₁₆X₁₇X₁₈RPS) consisting of the amino acid sequence as set forth in SEQ ID NO: 64, X₁₅ to X₁₈ each may be selected from acidic, neutral, and basic amino acids. Specifically, X₁₅ and X₁₆ each may be an acidic or neutral amino acid, and X₁₇ may be a neutral amino acid, and X₁₈ may be a basic or neutral amino acid. For example, X₁₅ to X₁₈ each may be selected from the group consisting of tyrosine, alanine, aspartic acid, serine, asparagine, histidine (His), glutamine (Gln), and lysine. Specifically, X₁₅ may be tyrosine, alanine, aspartic acid, or serine; X₁₆ may be aspartic acid or asparagine; X₁₇ may be serine or asparagine; and X₁₈ may be histidine, glutamine, or lysine. In an embodiment of the present disclosure, the light chain CDR2 may consist of an amino acid sequence as set forth in SEQ ID NO: 44, 47, 50, 53, 56, or 59.

In the light chain CDR3 (X₁₉X₂₀WDX₂₁SLX₂₂X₂₃YV) consisting of the amino acid sequence as set forth in SEQ ID NO: 65, X₁₉ to X₂₃ each may be selected from neutral amino acids. For example, X₁₉ to X₂₃ each may be selected from the group consisting of glycine, alanine, serine, threonine, tyrosine, aspartic acid, and asparagine. Specifically, X₁₉ may be glycine or alanine; X₂₀ may be alanine, serine, or threonine; X₂₁ may be tyrosine, serine, alanine, or aspartic acid; X₂₂ may be serine or asparagine; and X₂₃ may be alanine or glycine. In an embodiment of the present disclosure, the light chain CDR3 may consist of an amino acid sequence as set forth in SEQ ID NO: 45, 48, 51, 54, 57, or 60.

In another aspect of the present disclosure, the antibody or antigen-binding fragment thereof may include: a heavy chain variable region including, a heavy chain CDR1 selected from the group consisting of an amino acid sequence as set forth in SEQ ID NOS: 25, 28, 31, 34, 37, and 40, respectively, a heavy chain CDR2 selected from the group consisting of amino acid sequences as set forth in SEQ ID NOS: 26, 29, 32, 35, 38, and 41, respectively, and a heavy chain CDR3 selected from the group consisting of amino acid sequences as set forth in SEQ ID NOS: 27, 30, 33, 36, 39, and 42, respectively; and a light chain variable region including, a light chain CDR1 selected from the group consisting of amino acid sequences as set forth in SEQ ID NOS: 43, 46, 49, 52, 55, and 58, respectively, a light chain CDR2 selected from the group consisting of amino acid sequences as set forth in SEQ ID NOS: 44, 47, 50, 53, 56, and 59, respectively, and a light chain CDR3 selected from the group consisting of amino acid sequences as set forth in SEQ ID NOS: 45, 48, 51, 54, 57, and 60, respectively.

In addition, the antibody or antigen-binding fragment thereof may include: a heavy chain variable region selected from the group composing of, a heavy chain variable region including heavy chain CDR1, CDR2, and CDR3 consisting of the amino acid sequences as set forth in SEQ ID NOS: 25, 26, and 27, respectively, a heavy chain variable region including heavy chain CDR1, CDR2, and CDR3 consisting of the amino acid sequences as set forth in SEQ ID NOS: 28, 29, and 30, respectively, a heavy chain variable region including heavy chain CDR1, CDR2, and CDR3 consisting of the amino acid sequences as set forth in SEQ ID NOS: 31, 32, and 33, respectively, a heavy chain variable region including heavy chain CDR1, CDR2, and CDR3 consisting of the amino acid sequences as set forth in SEQ ID NOS: 34, 35, and 36, respectively, a heavy chain variable region including heavy chain CDR1, CDR2, and CDR3 consisting of the amino acid sequences as set forth in SEQ ID NOS: 37, 38, and 39, respectively, and a heavy chain variable region including heavy chain CDR1, CDR2, and CDR3 consisting of the amino acid sequences as set forth in SEQ ID NOS: 40, 41, and 42, respectively; and a light chain variable region selected from the group composing of, a light chain variable region including light chain CDR1, CDR2, and CDR3 consisting of the amino acid sequences as set forth in SEQ ID NOS: 43, 44, and 45, respectively, a light chain variable region including light chain CDR1, CDR2, and CDR3 consisting of the amino acid sequences as set forth in SEQ ID NOS: 46, 47, and 48, respectively, a light chain variable region including light chain CDR1, CDR2, and CDR3 consisting of the amino acid sequences as set forth in SEQ ID NOS: 49, 50, and 51, respectively, a light chain variable region including light chain CDR1, CDR2, and CDR3 consisting of the amino acid sequences as set forth in SEQ ID NOS: 52, 53, and 54, respectively, a light chain variable region including light chain CDR1, CDR2, and CDR3 consisting of the amino acid sequences as set forth in SEQ ID NOS: 55, 56, and 57, respectively, and a light chain variable region including light chain CDR1, CDR2, and CDR3 consisting of the amino acid sequences as set forth in SEQ ID NOS: 58, 59, and 60, respectively.

Furthermore, the antibody or antigen-binding fragment thereof may include: a heavy chain variable region including heavy chain CDR1, CDR2, and CDR3 consisting of the amino acid sequences as set forth in SEQ ID NOS: 25, 26, and 27, respectively, and a light chain variable region including light chain CDR1, CDR2, and CDR3 consisting of the amino acid sequences as set forth in SEQ ID NOS: 43, 44, and 45, respectively; a heavy chain variable region including heavy chain CDR1, CDR2, and CDR3 consisting of the amino acid sequences as set forth in SEQ ID NOS: 28, 29, and 30, respectively, and a light chain variable region including light chain CDR1, CDR2, and CDR3 consisting of the amino acid sequences as set forth in SEQ ID NOS: 46, 47, and 48, respectively; a heavy chain variable region including heavy chain CDR1, CDR2, and CDR3 consisting of the amino acid sequences as set forth in SEQ ID NOS: 31, 32, and 33, respectively, and a light chain variable region including light chain CDR1, CDR2, and CDR3 consisting of the amino acid sequences as set forth in SEQ ID NOS: 49, 50, and 51, respectively; a heavy chain variable region including heavy chain CDR1, CDR2, and CDR3 consisting of the amino acid sequences as set forth in SEQ ID NOS: 34, 35, and 36, respectively, and a light chain variable region including light chain CDR1, CDR2, and CDR3 consisting of the amino acid sequences as set forth in SEQ ID NOS: 52, 53, and 54, respectively; a heavy chain variable region including heavy chain CDR1, CDR2, and CDR3 consisting of the amino acid sequences as set forth in SEQ ID NOS: 37, 38, and 39, respectively, and a light chain variable region including light chain CDR1, CDR2, and CDR3 consisting of the amino acid sequences as set forth in SEQ ID NOS: 55, 56, and 57, respectively; or a heavy chain variable region including heavy chain CDR1, CDR2, and CDR3 consisting of the amino acid sequences as set forth in SEQ ID NOS: 40, 41, and 42, respectively, and a light chain variable region including light chain CDR1, CDR2, and CDR3 consisting of the amino acid sequences as set forth in SEQ ID NOS: 58, 59, and 60, respectively.

For example, the heavy chain variable region may be a polypeptide consisting of an amino acid sequence as set forth in SEQ ID NO: 1, 3, 5, 7, 9, or 11, and the light chain variable region may be a polypeptide consisting of an amino acid sequence as set forth in SEQ ID NO: 13, 15, 17, 19, 21, or 23.

In addition, the antibody or antigen-binding fragment thereof according to the present disclosure may be modified as needed. Specifically, the antibody or antigen-binding fragment thereof may be modified by conjugation, glycosylation, tagging, or a combination thereof. Specifically, the antibody or antigen-binding fragment thereof may be modified with horseradish peroxidase (HRP), an alkaline phosphatase, hapten, biotin, streptavidin, a fluorescent substance, a radioactive substance, a quantum dot, polyethylene glycol (PEG), a histidine tag, or the like. The antibody or antigen-binding fragment thereof may also be conjugated to another drug as needed.

The antibody or antigen-binding fragment thereof may be produced according to a monoclonal antibody production method known in the art, and the production method may be appropriately modified by a person skilled in the art. For example, the antibody may be produced by preparing hybridomas through the use of B lymphocytes obtained from an animal immunized with an antigen, or may be produced by phage display technology.

Furthermore, the present disclosure provides a nucleic acid encoding the antibody or antigen-binding fragment thereof.

An antibody or antigen-binding fragment thereof encoded by the nucleic acid according to the present disclosure may have the features as described above. As long as an amino acid sequence constituting the antibody or antigen-binding fragment thereof according to the present disclosure is known, a nucleic acid encoding the same would also be obvious to a person skilled in the art. The nucleic acid sequence may have at least one nucleotide addition, deletion, or substitution as long as the activity of the antibody or antigen-binding fragment translated thereof is retained.

For example, a nucleic acid encoding a heavy chain variable region included in the antigen or antigen-binding fragment thereof according to the present disclosure may be a polynucleotide consisting of a nucleotide sequence as set forth in SEQ ID NO: 2, 4, 6, 8, 10, or 12; and a nucleic acid encoding a light chain variable region included therein may be a polynucleotide consisting of a nucleotide sequence as set forth in SEQ ID NO: 14, 16, 18, 20, 22, or 24.

Furthermore, the present disclosure provides an expression vector including the nucleic acid.

The nucleic acid included in the expression vector according to the present disclosure may encode the antibody or antigen-binding fragment thereof having the features as described above.

As used herein, the term "expression vector" is a means for expressing a target gene in a host cell, and may include all of a plasmid vector, a cosmid vector, a bacteriophage vector, a viral vector, and the like. The expression vector may include elements necessary for preparing the peptide from the nucleic acid included therein. Specifically, the expression vector may include a signal sequence, an origin of replication, a marker gene, a promoter, a transcription termination sequence, and the like. The nucleic acid encoding the antibody or antigen-binding fragment thereof according to the present disclosure may be operatively linked to the promoter.

As an example, an expression vector used in prokaryotic cells may include a promoter for transcription, a ribosome binding site for initiation of translation, and termination sequences for transcription and translation. The expression vector used in eukaryotic cells may include a promoter and polyadenylation sequence derived from a mammal or a mammalian virus.

All marker genes known in the art may be used as a marker gene included in the expression vector, and may be specifically an antibiotic resistant gene. Specifically, the antibiotic resistant gene may be a gene showing resistance to antibiotics including ampicillin, gentamicin, carbenicillin, chloramphenicol, streptomycin, kanamycin, neomycin, tetracycline, and the like.

In addition, the present disclosure provides a host cell including the nucleic acid or the expression vector.

The nucleic acid or the expression vector included in the host cell according to the present disclosure may have the features as described above. As an example, the nucleic acid can encode the antibody or antigen-binding fragment thereof that specifically binds to ASM protein according to the present disclosure, and the expression vector may include the nucleic acid as described above.

All types of cells known to be usable to produce antibodies or antigen-binding fragments thereof in the art may be used as a host cell. Specifically, the host cell may be a prokaryotic cell, yeast, or a eukaryotic cell. Examples of the prokaryotic cell may include *E. coli,* the genus Bacillus strains, the genus Streptomyces strain, the genus Pseudomonas strain, the genus Staphylococcus strain, and the like, and examples of the yeast may include Saccharomyces cerevisiae and the like. Example of the eukaryotic cell may include COS-7, BHK, CHO, CHOK1, DXB-11, DG-44, CHO/-DHFR, CV1, HEK293, TM4, VERO, HELA, MDCK, BRL 3A, W138, Hep G2, SK-Hep, MMT, TRI, MRC 5, FS4, 3T3, RIN, A549, PC12, K562, PERC6, SP2/0, NS-0, U20S, HT1080, and the like.

The host cell may be transduced with the nucleic acid or expression vector as described above according to a method known in the art. Specifically, the transduction may be performed by transient transfection, microinjection, transduction, cell infusion, calcium phosphate precipitation, liposome-mediated transfection, DEAE dextran-mediated transfection, polybrene-mediated transfection, electroporation, a gene gun, or the like. The methods may also be appropriately modified by a person skilled in the art.

Furthermore, the present disclosure provides a method for producing an antibody or antigen-binding fragment thereof that specifically binds to an ASM protein, the method including culturing the host cell to produce the antibody or antigen-binding fragment thereof.

An antibody or antigen-binding fragment thereof obtained by the production method according to the present disclosure may have the features as described above.

The culture may be performed using an appropriate culture medium depending on the type of host cell used for production, and appropriate supplements may be contained therein as necessary. The culture may be performed in an appropriate environment depending on the type of host cell.

The production method according to the present disclosure may further include recovering an antibody or antigen-binding fragment thereof produced in the host cell. The recovering may be performed according to a method known in the art, wherein the culture may be modified as needed by a person skilled in the art. For example, the recovery may be performed by removing impurities through centrifugation or ultrafiltration, and may be performed by further purifying the obtained resultant product by chromatography or the like. The chromatography may include affinity chromatography, cation chromatography, hydrophobic interaction chromatography, and the like.

Furthermore, the present disclosure provides a composition and kit for detecting an ASM protein, each including the antibody or antigen-binding fragment thereof.

The antibody or antigen-binding fragment thereof included in the composition and kit for detecting an ASM protein according to the present disclosure may have the features as described above.

Further, the composition may include a ligand that can specifically bind to the antibody or antigen-binding fragment thereof according to the present disclosure. The ligand may be a conjugate labeled with a detector, such as a chromogenic enzyme, a fluorescent substance, a radioisotope, a colloid, or the like, or may be treated with streptavidin or avidin. The detecting composition of the present disclosure may further include, in addition to the reagents as described above, distilled water or a buffer to stably maintain the structures thereof.

The kit may be bound to a solid substrate to facilitate subsequent steps, such as washing of the antibody or antigen-binding fragment thereof contained therein, or separation of the complex. A synthetic resin, nitrocellulose, a glass substrate, a metal substrate, a glass fiber, a microsphere, or a micro-bead may be used as a solid substrate. In addition, polyester, polyvinyl chloride, polystyrene, polypropylene, PVDF or nylon may be used as a synthetic resin.

The kit may be manufactured by a conventional manufacturing method known to a person skilled in the art, and may further include a buffer, a stabilizer, an inactive protein, and the like.

Furthermore, the present disclosure provides a method for detecting an ASM protein, the method including a step of reacting a sample with the antibody or antigen-binding fragment thereof.

An antibody or antigen-binding fragment thereof used in the ASM protein detection method according to the present disclosure may have the features as described above.

The sample may include all kinds of samples that are used to detect the ASM protein. A method for detecting a target protein by using an antibody or antigen-binding fragment thereof is well known in the art, and the method may be performed by a person skilled in the art through appropriate modification as needed.

Hereinafter, the present disclosure will be described in detail with reference to the following examples. However, the following exemplary embodiments are merely for illustrating the present disclosure, and are not intended to limit the scope of the present disclosure. Any embodiment that has substantially the same constitution as the technical idea described in the claims of the disclosure and achieves the same effects of action should be included in the technical scope of the present disclosure.

### Example 1: Production of Antibodies Specifically Binding to Acid Sphingomyelinase (ASM) Protein

Antibodies that specifically bind to human ASM protein (SEQ ID NO: 66) and mouse ASM protein (SEQ ID NO: 67) were produced as follows.

Specifically, phages with scFv specifically binding to the human ASM protein or mouse ASM protein were selected by performing panning through a typical method using a recombinant human and mouse ASM protein and the human synthetic scFv-phage library. Nucleic acid sequences encoding the selected scFv were analyzed, and amino acid sequences translated therefrom were identified. Expression vectors were produced such that a mouse heavy chain constant region and a mouse light chain λ constant region consisting of the nucleotide sequences as set forth in SEQ ID NOS: 75 and 77, respectively, were linked to the carboxy termini of the heavy chain variable region and the light chain variable region of each of the identified scFv sequences. The amino acid sequences and nucleic acid sequences of the heavy chain variable regions constituting scFv contained in the produced expression vectors are shown in Table 1, and the amino acid sequences and nucleic acid sequences of the light chain variable regions thereof are disclosed in Table 2.

**TABLE 1**

| Antibody # | Sequence | Sequence Number |
|---|---|---|
| #9101 | EVQLLESGGGLVQPGGSLRLSCAASGFTFSNYAMSWVRQAP GKGLEWVSGIYPNGGNKYYADSVKGRFTISRDNSKNTLYLQ MNSLRAEDTAVYYCAKNAYRFDYWGQGTLVTVSS | SEQ ID NO: 1 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATGCTATGAGCTGGGTCCGCCAGGCTCCA GGGAAGGGGCTGGAGTGGGTCTCAGGGATCTATCCTAATGG TGGTAATAAATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAAAATGCTTATCGTTTCGACTACTGGGGCCAGGGTA CACTGGTCACCGTGAGCTCA | SEQ ID NO: 2 |
| #9102 | EVQLLESGGGLVQPGGSLRLSCAASGFTFSGYYMSWVRQAP GKGLEWVSLISPGSGSIYYADSVKGRFTISRDNSKNTLYLQ MNSLRAEDTAVYYCAKSWHHFDYWGQGTLVTVSS | SEQ ID NO: 3 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCGGTTATTATATGAGCTGGGTCCGCCAGGCTCCA GGGAAGGGGCTGGAGTGGGTCTCATTGATCTCTCCTGGTAG TGGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAATCTTGGCATCATTTCGACTACTGGGGCCAGGGTA | SEQ ID NO: 4 |
| | CACTGGTCACCGTGAGCTCA | |
| #9104 | EVQLLESGGGLVQPGGSLRLSCAASGFTFSNYYMSWVRQAP GKGLEWVSGIYYGSGNIYYADSVKGRFTISRDNSKNTLYLQ MNSLRAEDTAVYYCARDTPGFDYWGQGTLVTVSS | SEQ ID NO: 5 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTAG TGGTAATATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 6 |
| #9108 | EVQLLESGGGLVQPGGSLRLSCAASGFTFSNYAMSWVRQAP GKGLEWVSAIYPGGGSIYYADSVKGRFTISRDNSKNTLYLQ MNSLRAEDTAVYYCARDVLGLTPKPFDYWGQGTLVTVSS | SEQ ID NO: 7 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATGCTATGAGCTGGGTCCGCCAGGCTCCA GGGAAGGGGCTGGAGTGGGTCTCAGCGATCTATCCTGGTGG TGGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATGTTTTGGGTCTGACTCCTAAGCCGTTCGACT ACTGGGGCCAGGGTACACTGGTCACCGTGAGCTCA | SEQ ID NO: 8 |
| #9113 | EVQLLESGGGLVQPGGSLRLSCAASGFTFSSYYMSWVRQAP GKGLEWVSSISPGGSSIYYADSVKGRFTISRDNSKNTLYLQ MNSLRAEDTAVYYCAKGASLFDYWGQGTLVTVSS | SEQ ID NO: 9 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAGTTATTATATGAGCTGGGTCCGCCAGGCTCCA GGGAAGGGGCTGGAGTGGGTCTCATCGATCTCTCCTGGTGG TAGTAGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAAAGGGGCGTCTCTGTTCGACTACTGGGGCCAGGGTA CACTGGTCACCGTGAGCTCA | SEQ ID NO: 10 |
| #9123 | EVQLLESGGGLVQPGGSLRLSCAASGFTFSDYAMSWVRQAP GKGLEWVSAISYGGGNIYYADSVKGRFTISRDNSKNTLYLQ MNSLRAEDTAVYYCARVGGMCTRRQCYYDYGMDVWGQGTLV TVSS | SEQ ID NO: 11 |
| | GAGGTGCAGCTGTTGGAGTCTGGGGGAGGCTTGGTACAGCC | SEQ ID NO: 12 |
| | TGGGGGGTCCCTGAGACTCTCCTGTGCAGCCTCCGGATTCA CCTTTAGCGATTATGCTATGAGCTGGGTCCGCCAGGCTCCA GGGAAGGGGCTGGAGTGGGTCTCAGCGATCTCTTATGGTGG TGGTAATATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGTTGGTGGTATGTGTACTAGGCGTCAGTGTTATT ATGATTATGGTATGGACGTCTGGGGCCAGGGTACACTGGTC ACCGTGAGCTCA | |

**TABLE 2**

| Antibody # | Sequence | SEQ ID NO |
|---|---|---|
| #9101 | QSVLTQPPSASGTPGQRVTISCSGSSSNIGNNYVSWYQQLPG TAPKLLIYADSKRPSGVPDRFSGSKSGTSASLAIGGLRSEDE ADYYCGSWDYSLNAYVFGGGTKLTVL | SEQ ID NO: 13 |
| | CAGTCTGTGCTGACTCAGCCACCCTCAGCTAGCGGGACCCCC GGGCAGAGGGTCACCATCTCTTGTAGTGGCTCTTCATCCAAT ATTGGCAATAATTATGTCTCCTGGTACCAGCAGCTCCCAGGA ACGGCCCCCAAACTCCTCATCTATGCTGATAGTAAGCGGCCA AGCGGGGTCCCTGACCGATTCTCTGGCTCCAAGTCTGGCACC TCAGCCTCGCTGGCCATCGGTGGGCTCCGGTCCGAGGATGAG GCTGATTATTACTGTGGTTCTTGGGATTATAGCCTGAATGCT TATGTCTTCGGCGGAGGCACCAAGCTTACGGTCCTA | SEQ ID NO: 14 |
| #9102 | QSVLTQPPSASGTPGQRVTISCSGSSSNIGNNPVYWYQQLPG TAPKLLIYANNQRPSGVPDRFSGSKSGTSASLAISGLRSEDE ADYYCAAWDSSLSGYVFGGGTKLTVL | SEQ ID NO: 15 |
| | CAGTCTGTGCTGACTCAGCCACCCTCAGCTAGCGGGACCCCC GGGCAGAGGGTCACCATCTCTTGTAGTGGCTCTTCATCTAAT ATTGGCAATAATCCTGTCTACTGGTACCAGCAGCTCCCAGGA ACGGCCCCCAAACTCCTCATCTATGCTAATAATCAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGCTGCTTGGGATTCTAGCCTGAGTGGT TATGTCTTCGGCGGAGGCACCAAGCTTACGGTCCTA | SEQ ID NO: 16 |
| #9104 | QSVLTQPPSASGTPGQRVTISCTGSSSNIGNNAVNWYQQLPG TAPKLLIYYDSHRPSGVPDRFSGSKSGTSASLAISGLRSEDE ADYYCGAWDYSLSAYVFGGGTKLTVL | SEQ ID NO: 17 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA | SEQ ID NO: 18 |
| | ACGGCCCCCAAACTCCTCATCTATTATGATAGTCATCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | |
| #9108 | QSVLTQPPSASGTPGQRVTLSCTGSSSNIGSNTVYWYQQLPG TAPKLLIYANSQRPSGVPDRFSGSKSGTSASLAISGLRSEDE ADYYCGSWDYSLSGYVFGGGTKLTVL | SEQ ID NO: 19 |
| | CAGTCTGTGCTGACTCAGCCACCCTCAGCTAGCGGGACCCCC GGGCAGAGGGTCACCCTCTCTTGTACTGGCTCTTCATCTAAT ATTGGCAGTAATACTGTCTACTGGTACCAGCAGCTCCCAGGA ACGGCCCCCAAACTCCTCATCTATGCTAATAGTCAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTTCTTGGGATTATAGCCTGAGTGGT TATGTCTTCGGCGGAGGCACCAAGCTTACGGTCCTA | SEQ ID NO: 20 |
| #9113 | QSVLTQPPSASGTPGQRVTISCSGSSSNIGNNAVSWYQQLPG TAPKLLIYSDNKRPSGVPDRFSGSKSGTSASLAISGLRSEDE ADYYCGTWDASLNAYVFGGGTKLTVL | SEQ ID NO: 21 |
| | CAGTCTGTGCTGACTCAGCCACCCTCAGCTAGTGGGACCCCC GGGCAGAGGGTCACCATCTCTTGTAGTGGCTCTTCATCTAAT ATTGGCAATAATGCTGTCTCCTGGTACCAGCAGCTCCCAGGA ACGGCCCCCAAACTCCTCATCTATTCTGATAATAAGCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTACTTGGGATGCTAGCCTGAATGCT TATGTCTTCGGCGGAGGCACCAAGCTTACGGTCCTA | SEQ ID NO: 22 |
| #9123 | QSVLTQPPSASGTPGQRVTISCSGSSSNIGSNTVNWYQQLPG TAPKLLIYDNSKRPSGVPDRFSGSKSGTSASLAISGLRSEDE ADYYCGTWDDSLSGYVFGGGTKLTVL | SEQ ID NO: 23 |
| | CAGTCTGTGCTGACTCAGCCACCCTCAGCTAGCGGGACCCCC GGGCAGAGGGTCACCATCTCTTGTAGTGGCTCTTCATCTAAT ATTGGCAGTAATACTGTCAACTGGTACCAGCAGCTCCCAGGA ACGGCCCCCAAACTCCTCATCTATGATAATAGTAAGCGGCCA AGCGGGGTCCCTGACCGATTCTCTGGCTCCAAGTCTGGCACC TCAGCCTCCCTGGCCATCAGTGGGCTCCGGTCCGAAGATGAG GCTGATTATTACTGTGGTACTTGGGATGATAGCCTGAGTGGT TATGTCTTCGGCGGAGGCACCAAGCTTACGGTCCTA | SEQ ID NO: 24 |

The expression vector employed a mammalian expression vector. The produced expression vectors were transformed into the CHO or HEK293 cell line to produce full-length antibodies binding to human ASM protein, or to both human and mouse ASM proteins.

### Example 2: Determination of Complementarity Determining Regions (CDRs)

The complementarity determining regions in the produced scFv fragments were identified by a conventional method, and as a result, the CDR sequences of the heavy chain variable regions are shown in Table 3 and the CDR sequences of the light chain variable regions are shown in Table 4.

**TABLE 3**

| Antibody # | CDR | Sequence | SEQ ID NO |
|---|---|---|---|
| #9101 | CDR1 | NYAMS | SEQ ID NO: 25 |
| | CDR2 | GIYPNGGNKYYADSVKG | SEQ ID NO: 26 |
| | CDR3 | NAYRFDY | SEQ ID NO: 27 |
| #9102 | CDR1 | GYYMS | SEQ ID NO: 28 |
| | CDR2 | LISPGSGSIYYADSVKG | SEQ ID NO: 29 |
| | CDR3 | SWHHFDY | SEQ ID NO: 30 |
| #9104 | CDR1 | NYYMS | SEQ ID NO: 31 |
| | CDR2 | GIYYGSGNIYYADSVKG | SEQ ID NO: 32 |
| | CDR3 | DTPGFDY | SEQ ID NO: 33 |
| #9108 | CDR1 | NYAMS | SEQ ID NO: 34 |
| | CDR2 | AIYPGGGSIYYADSVKG | SEQ ID NO: 35 |
| | CDR3 | DVLGLTPKPFDY | SEQ ID NO: 36 |
| #9113 | CDR1 | SYYMS | SEQ ID NO: 37 |
| | CDR2 | SISPGGSSIYYADSVKG | SEQ ID NO: 38 |
| | CDR3 | GASLFDY | SEQ ID NO: 39 |
| #9123 | CDR1 | DYAMS | SEQ ID NO: 40 |
| | CDR2 | AISYGGGNIYYADSVKG | SEQ ID NO: 41 |
| | CDR3 | VGGMCTRRQCYYDYGMDV | SEQ ID NO: 42 |

**TABLE 4**

| Antibody # | CDR | Sequence | SEQ ID NO |
|---|---|---|---|
| #9101 | CDR1 | SGSSSNIGNNYVS | SEQ ID NO: 43 |
| | CDR2 | ADSKRPS | SEQ ID NO: 44 |
| | CDR3 | GSWDYSLNAYV | SEQ ID NO: 45 |
| #9102 | CDR1 | SGSSSNIGNNPVY | SEQ ID NO: 46 |
| | CDR2 | ANNQRPS | SEQ ID NO: 47 |
| | CDR3 | AAWDSSLSGYV | SEQ ID NO: 48 |
| #9104 | CDR1 | TGSSSNIGNNAVN | SEQ ID NO: 49 |
| | CDR2 | YDSHRPS | SEQ ID NO: 50 |
| | CDR3 | GAWDYSLSAYV | SEQ ID NO: 51 |
| #9108 | CDR1 | TGSSSNIGSNTVY | SEQ ID NO: 52 |
| | CDR2 | ANSQRPS | SEQ ID NO: 53 |
| | CDR3 | GSWDYSLSGYV | SEQ ID NO: 54 |
| #9113 | CDR1 | SGSSSNIGNNAVS | SEQ ID NO: 55 |
| | CDR2 | SDNKRPS | SEQ ID NO: 56 |
| | CDR3 | GTWDASLNAYV | SEQ ID NO: 57 |
| #9123 | CDR1 | SGSSSNIGSNTVN | SEQ ID NO: 58 |
| | CDR2 | DNSKRPS | SEQ ID NO: 59 |
| | CDR3 | GTWDDSLSGYV | SEQ ID NO: 60 |

### Experimental Example 1: Confirmation of Binding to ASM Protein

The binding of the produced antibodies, specifically binding to the ASM protein, to the ASM protein were analyzed by ELISA.

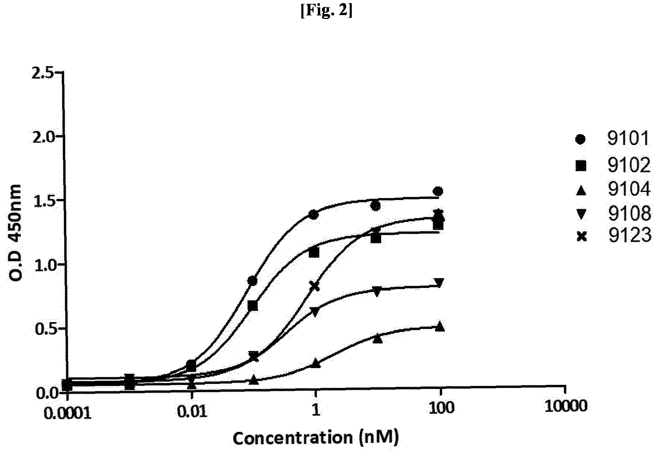

First, 100 µl of 1 pg/mL recombinant human ASM protein (R&D systems) was added to a multi-array 96-well plate (Thermo scientific) and left at 4°C for 16 hours to coat the plate. Then, 200 µl of PBS containing 5% BSA was added to the coated plate, followed by incubation at 37°C for about 2 hours, and then the plate was washed three times with PBS containing 0.05% Tween 20. The plate was treated with the anti-ASM antibody at 0.0001, 0.001, 0.01, 0.1, 1, 10, or 100 nM, followed by incubation at 37°C for about 1 hours. After the incubation, the plate was washed three times with PBS containing 0.05% Tween, and 100 ul of goat-anti-mouse IgG-HRP antibody (Jackson Immunoresearch) as a secondary antibody was added. After incubation again at 37°C for about 1 hour, the plate was washed three times with PBS containing 0.05% Tween. Then, 100 ul of a tetramethylbenzidine (TMB) substrate was added thereto, followed by incubation at room temperature for additional about 5 minutes, and then the reaction was terminated by 100 µl of a 2 N sulfuric acid solution, and the absorbance was measured at a wavelength of 450 nm. As a result, the measured absorbance was shown in FIG. 2 and the EC₅₀ values of antibodies calculated from the absorbance values are shown in Table 5.

**TABLE 5**

| Antibody # | EC₅₀ (nM) |
|---|---|
| #9101 | 0.0815 |
| #9102 | 0.0954 |
| #9104 | 1.948 |
| #9108 | 0.3269 |
| #9123 | 0.7578 |

As shown in FIG. 2, all of the five types of antibodies produced above were bound to the ASM protein in a concentration-dependent manner. In particular, as shown in Table 5, antibody #9101 showed the lowest EC₅₀ value and thus had the highest binding ability to ASM protein.

### Experimental Example 2: Confirmation of Binding Affinity with ASM Protein

The binding affinity of the produced antibodies, specifically binding to the ASM protein, with the ASM protein and the interactive dynamics therebetween were measured using an Octet^{®} QK384 system (Pall Life Sciences).

First, 20 mM 1-ethyl-3-(3-dimethylaminopropyl)carbodiimide hydrochloride (EDC) and 40 mM N-hydroxysulfosuccinimide (sulfo-NHS) solutions were treated on an AR2G sensor (ForteBio) to activate carboxyl groups therein. Meanwhile, the 10 ug/mL recombinant human ASM protein or 2.5 ug/mL recombinant mouse ASM protein was prepared by dilution in a 10 mM sodium acetate solution (pH 5.0) (ForteBio). The solution having the diluted ASM protein was added to the AR2G sensor having activated carboxyl groups to obtain the AR2G sensor in which the recombinant human or mouse ASM protein was immobilized. A 1 M ethanol amine (ForteBio) was added to the sensor in which the obtained ASM protein was immobilized, to inactivate the remaining unreacted carboxyl groups. The antibodies produced in Example 1 were added at concentrations of 0.4, 2, or 10 nM, and the association phase of the reaction product was observed for up to about 900 seconds. Thereafter, a 1× kinetics buffer (ForteBio) was added, and the dissociation phase of the reaction product was observed for about 1,200 seconds. The association constant (Kon), dissociation constant (Kdis), and equilibrium dissociation constant (KD) of each antibody were determined using Octet^{®} analysis software (Pall Life^{®} Sciences). As a result, the analysis results for the recombinant human ASM protein are shown in Table 6, and the analysis results for the recombinant mouse ASM protein are shown in Table 7.

**TABLE 6**

| Antibody # | KD (M) | Kon (1/Ms) | Kdis (1/s) | RMax | Full R² |
|---|---|---|---|---|---|
| #9101 | 1.16E-09 | 6.26E+05 | 7.28E-04 | 0.4527 | 0.9861 |
| #9102 | 2.52E-10 | 4.16E+05 | 1.05E-04 | 0.8652 | 0.997 |
| #9104 | 2.09E-10 | 3.92E+05 | 8.18E-05 | 0.3324 | 0.9759 |
| #9108 | 1.19E-10 | 2.49E+05 | 2.96E-05 | 0.45 | 0.999 |
| #9113 | 5.64E-10 | 1.16E+06 | 6.56E-04 | 0.2868 | 0.9635 |

**TABLE 7**

| Antibody # | KD (M) | Kon (1/Ms) | Kdis (1/s) | RMax | Full R² |
|---|---|---|---|---|---|
| #9101 | 4.25E-11 | 3.76E+06 | 1.59E-04 | 0.0185 | 0.5527 |
| #9102 | 4.98E-10 | 4.89E+05 | 2.43E-04 | 0.4281 | 0.9944 |
| #9104 | 6.38E-10 | 3.10E+05 | 1.98E-04 | 0.7027 | 0.9971 |
| #9108 | 3.39E-09 | 4.59E+05 | 1.56E-03 | 0.3921 | 0.9865 |
| #9113 | <1.0E-12 | 1.00E+06 | <1.0E-07 | 0 | 0 |

As shown in Table 6, five types of antibodies produced in Example 1 were bound to the human ASM protein with a binding affinity of 10⁻¹⁰ to 10⁻⁹ M. Meanwhile, as shown in Table 7, only the antibodies #9102, #9104, and #9108 showed a binding affinity of 10⁻¹⁰ to 10⁻⁹ M to the mouse ASM protein.

### Experimental Example 3: Identification of Antigenic Determinant of ASM Protein

The sites of the ASM protein to which the present inventive antibodies were bound, that is, antigenic determinants were investigated by hydrogen deuterium exchange mass spectrometry (HDX-MS).

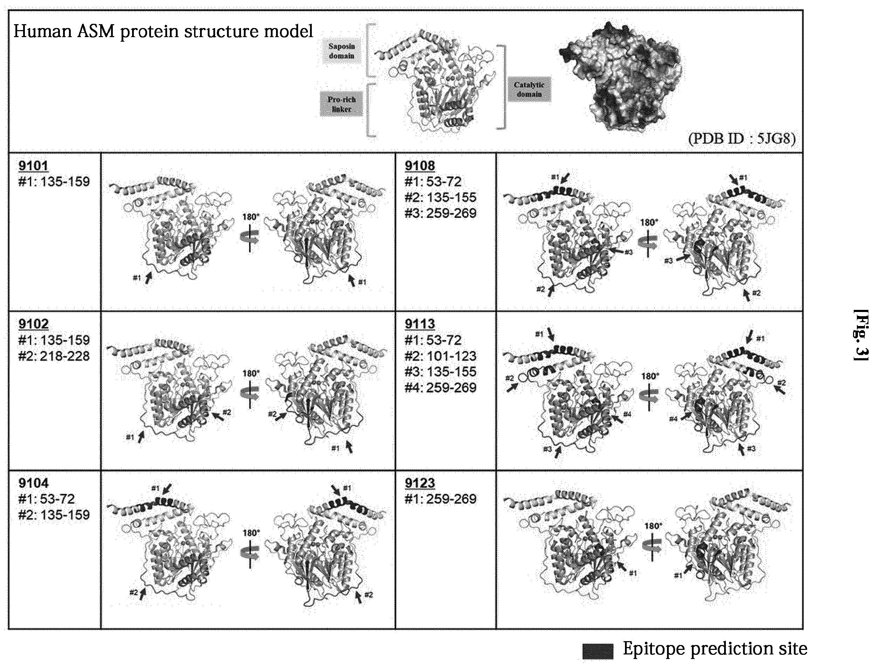

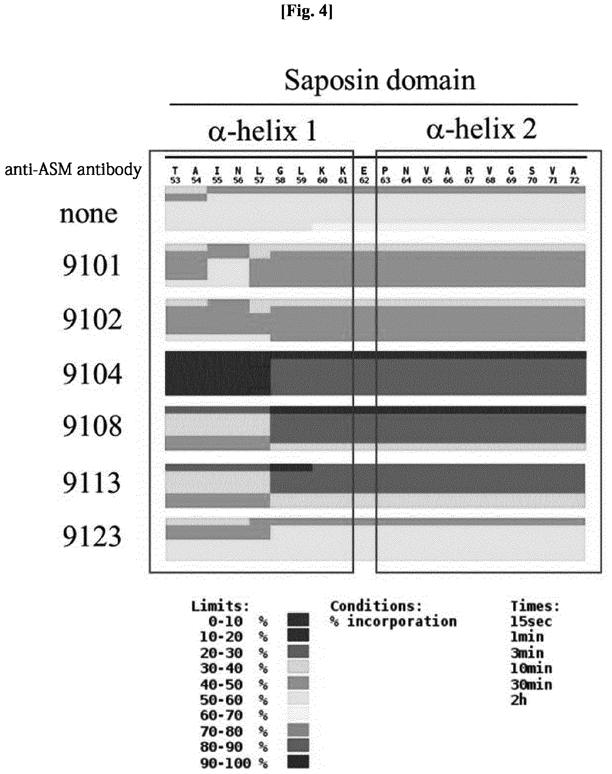

First, the 1,000 pg/mL ASM protein, 1,000 ug/mL #9101 mIgG1, 1,250 ug/mL #9102 mIgG2, 1,000 ug/mL #9104 mIgG4, 1,000 ug/mL #9108 mIgG1, 3,300 ug/mL #9113 mIgG1, or 2,932 ug/mL #9123 mIgG1 antibodies were serially diluted 2-fold to prepare up to 128-fold dilutions. Then, 1 µl of each of the prepared dilutions was taken, and mixed with an equal amount of 10 mg/ml sinapinic acid matrix (K200 MALDI kit, CovalX), and 1 µl of the mixture was dispensed in SCOUT 384 MALDI plates, followed by crystallization at room temperature. Thereafter, the crystals were measured using an MALDI mass spectrometer. For the cross-link data for non-covalent interaction analysis, 1 µl of each of the 128-fold dilutions was mixed with an equal amount of a 2 mg/ml K200 stabilizer reagent (K200 MALDI kit, CovalX), followed by incubation at room temperature for 3 hours. After the reaction was completed, 1 µl of the mixed solution was subjected to the measurement using an MALDI mass spectrometer, and the measurement results were analyzed in a high-mass MALDI MS mode. As a result, the peptide sequences of the sites that were identified to have significant difference of deuterium incorporation in the ASM protein are shown in Table 8, and human ASM protein structure models indicating the binding sites on ASM protein to each antibody are shown in FIG. 3. The deuterium exchange heat map of each antibody in the saposin domain of the ASM protein is shown in FIG. 4.

**TABLE 8**

| Antibody # | Peptide region | Amino acid sequence |
|---|---|---|
| #9101 | 135-159 | PTVPKPPPKPPSPPAPGAPVSRILF |
| #9102 | 135-159 | PTVPKPPPKPPSPPAPGAPVSRILF |
| | 218-228 | SGLGPAGPFDM |
| #9104 | 53-72 | TAINLGLKKEPNVARVGSVA |
| | 135-159 | PTVPKPPPKPPSPPAPGAPVSRILF |
| #9108 | 53-72 | TAINLGLKKEPNVARVGSVA |
| | 135-155 | PTVPKPPPKPPSPPAPGAPVS |
| | 259-269 | VRKFLGPVPVY |
| #9113 | 53-72 | TAINLGLKKEPNVARVGSVA |
| | 101-123 | VWRRSVLSPSEACGLLLGSTCGH |
| | 135-155 | PTVPKPPPKPPSPPAPGAPVS |
| | 259-269 | VRKFLGPVPVY |
| #9123 | 259-269 | VRKFLGPVPVY |

As shown in Table 8 and FIG. 3, the antibodies according to the present disclosure were bound to the fragment of amino acids at positions 53 to 72 (SEQ ID NO: 68, TAINLGLKKEPNVARVGSVA), the fragment of amino acids at positions 101 to 123 (SEQ ID NO: 69, VWRRSVLSPSEACGLLLGSTCGH), the fragment of amino acids at positions 135 to 159 (SEQ ID NO: 70, PTVPKPPPKPPSPPAPGAPVSRILF), the fragment of amino acids at positions 135 to 155 (SEQ ID NO: 71, PTVPKPPPKPPSPPAPGAPVS), the fragment of amino acid at positions 218 to 228 (SEQ ID NO: 72, SGLGPAGPFDM), or the fragment of amino acids at positions 259 to 269 (SEQ ID NO: 73, VRKFLGPVPVY) from the N-terminus of the ASM protein. Especially, as shown in FIG. 4, the antibodies according to the present disclosure were mainly bound to α-helix 1 and α-helix 2 in the saposin domain.

## Claims

1. An antibody or antigen-binding fragment thereof that specifically binds to an acid sphingomyelinase (ASM) protein.

2. The antibody or antigen-binding fragment thereof of claim 1, wherein the antibody or antigen-binding fragment thereof binds to at least one epitope selected from the group consisting of a fragment of amino acids at positions 53 to 72, a fragment of amino acids at positions 101 to 123, a fragment of amino acids at positions 135 to 159, a fragment of amino acids at positions 135 to 155, a fragment of amino acid at positions 218 to 228, and a fragment of amino acids at positions 259 to 269 from the N-terminus of the ASM protein.

3. The antibody or antigen-binding fragment thereof of claim 1, wherein the antibody or antigen-binding fragment thereof binds to at least one epitope selected from the group consisting of polypeptides as sets forth in SEQ ID NOS: 68 to 73, respectively.

4. The antibody or antigen-binding fragment thereof of claim 1, wherein the antibody or antigen-binding fragment thereof comprises:
a heavy chain variable region comprising a heavy chain CDR1 (X₁YX₂MS) consisting of the amino acid sequence as set forth in SEQ ID NO: 61, a heavy chain CDR2 (X₃IX₄X₅X₆X₇X₈X₉X₁₀YYADSVKG) consisting of the amino acid sequence as set forth in SEQ ID NO: 62, and a heavy chain CDR3 selected from the group consisting of amino acid sequences as set forth in SEQ ID NOS: 27, 30, 33, 36, 39, and 42; and
a light chain variable region comprising a light chain CDR1 (X₁₁GSSSNIGX₁₂NX₁₃VX₁₄) consisting of the amino acid sequence as set forth in SEQ ID NO: 63, a light chain CDR2 (X₁₅X₁₆X₁₇X₁₈RPS) consisting of the amino acid sequence as set forth in SEQ ID NO: 64, and a light chain CDR3 (X₁₉X₂₀WDX₂₁SLX₂₂X₂₃YV) consisting of the amino acid sequence as set forth in SEQ ID NO 65.

5. The antibody or antigen-binding fragment thereof of claim 4, wherein:
X₁ and X₂ each are selected from the group consisting of asparagine (Asn), glycine (Gly), serine (Ser), aspartic acid (Asp), alanine (Ala), and tyrosine (Tyr);
X₃ to X₁₀ each are selected from the group consisting of, glycine, leucine (Leu), alanine, serine, tyrosine, proline (Pro), asparagine, lysine (Lys), and isoleucine (Ile);
X₁₁ to X₁₄ each are selected from the group consisting of threonine (Thr), serine, asparagine, alanine, proline, and tyrosine;
X₁₅ to X₁₈ each are selected from the group consisting of tyrosine, alanine, aspartic acid, serine, asparagine, histidine (His), glutamine (Gln), and lysine; and
X₁₉ to X₂₃ each are selected from the group consisting of glycine, alanine, serine, threonine, tyrosine, aspartic acid, and asparagine.

6. The antibody or antigen-binding fragment thereof of claim 5, wherein:
X₁ is asparagine, glycine, serine, or aspartic acid;
X₂ is alanine or tyrosine;
X₃ is glycine, leucine, alanine, or serine;
X₄ is tyrosine or serine;
X₅ is proline or tyrosine;
X₆ is asparagine or glycine;
X₇ and X₈ each are glycine or serine;
X₉ is asparagine or serine;
X₁₀ is lysine or isoleucine;
X₁₁ is threonine or serine;
X₁₂ is asparagine or serine;
X₁₃ is alanine, proline, threonine, or tyrosine;
X₁₄ is asparagine, tyrosine, or serine;
X₁₅ is tyrosine, alanine, aspartic acid, or serine;
X₁₆ is aspartic acid or asparagine;
X₁₇ is serine or asparagine;
X₁₈ is histidine, glutamine, or lysine;
X₁₉ is glycine or alanine;
X₂₀ is alanine, serine, or threonine;
X₂₁ is tyrosine, serine, alanine, or aspartic acid;
X₂₂ is serine or asparagine; and
X₂₃ is alanine or glycine.

7. The antibody or antigen-binding fragment thereof of claim 1, wherein the antibody or antigen-binding fragment thereof comprises:
a heavy chain variable region comprising,
a heavy chain CDR1 selected from the group consisting of an amino acid sequence as set forth in SEQ ID NOS: 25, 28, 31, 34, 37, and 40 respectively,
a heavy chain CDR2 selected from the group consisting of amino acid sequences as set forth in SEQ ID NOS: 26, 29, 32, 35, 38, and 41, respectively, and
a heavy chain CDR3 selected from the group consisting of amino acid sequences as set forth in SEQ ID NOS: 27, 30, 33, 36, 39, and 42, respectively; and
a light chain variable region comprising,
a light chain CDR1 selected from the group consisting of amino acid sequences as set forth in SEQ ID NOS: 43, 46, 49, 52, 55, and 58, respectively,
a light chain CDR2 selected from the group consisting of amino acid sequences as set forth in SEQ ID NOS: 44, 47, 50, 53, 56, and 59, respectively, and
a light chain CDR3 selected from the group consisting of amino acid sequences as set forth in SEQ ID NOS: 45, 48, 51, 54, 57, and 60, respectively.

8. The antibody or antigen-binding fragment thereof of claim 1, wherein the antibody or antigen-binding fragment thereof comprises:
a heavy chain variable region selected from the group consisting of
a heavy chain variable region comprising heavy chain CDR1, CDR2, and CDR3 consisting of the amino acid sequences as set forth in SEQ ID NOS: 25, 26, and 27, respectively,
a heavy chain variable region comprising heavy chain CDR1, CDR2, and CDR3 consisting of the amino acid sequences as set forth in SEQ ID NOS: 28, 29, and 30, respectively,
a heavy chain variable region comprising heavy chain CDR1, CDR2, and CDR3 consisting of the amino acid sequences as set forth in SEQ ID NOS: 31, 32, and 33, respectively,
a heavy chain variable region comprising heavy chain CDR1, CDR2, and CDR3 consisting of the amino acid sequences as set forth in SEQ ID NOS: 34, 35, and 36, respectively,
a heavy chain variable region comprising heavy chain CDR1, CDR2, and CDR3 consisting of the amino acid sequences as set forth in SEQ ID NOS: 37, 38, and 39, respectively, and
a heavy chain variable region comprising heavy chain CDR1, CDR2, and CDR3 consisting of the amino acid sequences as set forth in SEQ ID NOS: 40, 41, and 42, respectively; and
a light chain variable region selected from the group consisting of
a light chain variable region comprising light chain CDR1, CDR2, and CDR3 consisting of the amino acid sequences as set forth in SEQ ID NOS: 43, 44, and 45, respectively,
a light chain variable region comprising light chain CDR1, CDR2, and CDR3 consisting of the amino acid sequences as set forth in SEQ ID NOS: 46, 47, and 48, respectively,
a light chain variable region comprising light chain CDR1, CDR2, and CDR3 consisting of the amino acid sequences as set forth in SEQ ID NOS: 49, 50, and 51, respectively,
a light chain variable region comprising light chain CDR1, CDR2, and CDR3 consisting of the amino acid sequences as set forth in SEQ ID NOS: 52, 53, and 54, respectively,
a light chain variable region comprising light chain CDR1, CDR2, and CDR3 consisting of the amino acid sequences as set forth in SEQ ID NOS: 55, 56, and 57, respectively, and
a light chain variable region comprising light chain CDR1, CDR2, and CDR3 consisting of the amino acid sequences as set forth in SEQ ID NOS: 58, 59, and 60, respectively.

9. The antibody or antigen-binding fragment thereof of claim 1, wherein the antibody or antigen-binding fragment thereof comprises:
a heavy chain variable region comprising heavy chain CDR1, CDR2, and CDR3 consisting of the amino acid sequences as set forth in SEQ ID NOS: 25, 26, and 27, respectively, and a light chain variable region comprising light chain CDR1, CDR2, and CDR3 consisting of the amino acid sequences as set forth in SEQ ID NOS: 43, 44, and 45, respectively;
a heavy chain variable region comprising heavy chain CDR1, CDR2, and CDR3 consisting of the amino acid sequences as set forth in SEQ ID NOS: 28, 29, and 30, respectively, and a light chain variable region comprising light chain CDR1, CDR2, and CDR3 consisting of the amino acid sequences as set forth in SEQ ID NOS: 46, 47, and 48, respectively;
a heavy chain variable region comprising heavy chain CDR1, CDR2, and CDR3 consisting of the amino acid sequences as set forth in SEQ ID NOS: 31, 32, and 33, respectively, and a light chain variable region comprising light chain CDR1, CDR2, and CDR3 consisting of the amino acid sequences as set forth in SEQ ID NOS: 49, 50, and 51, respectively;
a heavy chain variable region comprising heavy chain CDR1, CDR2, and CDR3 consisting of the amino acid sequences as set forth in SEQ ID NOS: 34, 35, and 36, respectively, and a light chain variable region comprising light chain CDR1, CDR2, and CDR3 consisting of the amino acid sequences as set forth in SEQ ID NOS: 52, 53, and 54, respectively;
a heavy chain variable region comprising heavy chain CDR1, CDR2, and CDR3 consisting of the amino acid sequences as set forth in SEQ ID NOS: 37, 38, and 39, respectively, and a light chain variable region comprising light chain CDR1, CDR2, and CDR3 consisting of the amino acid sequences as set forth in SEQ ID NOS: 55, 56, and 57, respectively; or
a heavy chain variable region comprising heavy chain CDR1, CDR2, and CDR3 consisting of the amino acid sequences as set forth in SEQ ID NOS: 40, 41, and 42, respectively, and a light chain variable region comprising light chain CDR1, CDR2, and CDR3 consisting of the amino acid sequences as set forth in SEQ ID NOS: 58, 59, and 60, respectively.

10. The antibody or antigen-binding fragment thereof of claim 1, wherein the ASM protein is derived from a mammal.

11. The antibody or antigen-binding fragment thereof of claim 1, wherein the ASM protein is a polypeptide consisting of an amino acid sequence as set forth in SEQ ID NO: 66 or 67.

12. A nucleic acid encoding the antibody or antigen-binding fragment thereof of claim 1.

13. An expression vector comprising the nucleic acid of claim 12.

14. A host cell comprising the nucleic acid of claim 12 or the expression vector of claim 13.

15. A method for producing an antibody or antigen-binding fragment thereof that specifically binds to an ASM protein, the method comprising culturing the host cell of claim 14 to produce the antibody or antigen-binding fragment thereof.

16. A composition for detecting an ASM protein, the composition comprising the antibody or antigen-binding fragment thereof of any one of claims 1 to 11.

17. A kit for detecting an ASM protein, the kit comprising the antibody or antigen-binding fragment thereof of any one of claims 1 to 11.

18. A method for detecting an ASM protein, the method comprising reacting a sample with the antibody or antigen-binding fragment thereof of any one of claims 1 to 11.
